# EUROPEAN PATENT SPECIFICATION

(11) **EP 4 424 261 B1**
(45) Date of publication and mention of the grant of the patent: **18.02.2026**
(21) Application number: 21963159.5
(22) Date of filing: 30.12.2021
(51) Int. Cl.: A61B 18/20

(54) **HANDHELD HOME LASER HAIR REMOVAL DEVICE AND SYSTEM**
TRAGBARES HAUSHALTSLASERHAARENTFERNUNGSINSTRUMENT UND SYSTEM
INSTRUMENT PORTATIF D'ÉPILATION LASER ET SON SYSTÈME

(30) Priority: 02.11.2021 CN 202111286379; 02.11.2021 CN 202122653783 U
(43) Date of publication of application: 04.09.2024
(73) Proprietor: Wuhan Lotuxs Technology Co., Ltd., Wuhan, Hubei 430206 (CN)
(72) Inventor: YANG, Lin, Wuhan, Hubei 430206 (CN)
(74) Representative: Canzler & Bergmeier Patentanwälte Partnerschaft mbB
(86) International application number: PCT/CN2021/143096
(87) International publication number: WO 2023/077653

(56) References cited:
- CN-A- 109 620 401
- CN-A- 111 166 469
- CN-A- 111 166 469
- CN-A- 113 509 261
- CN-U- 204 275 310
- CN-U- 214 180 573
- CN-U- 214 180 573
- CN-Y- 2 928 026
- US-A1- 2009 204 109
- US-A1- 2016 287 333
- US-A1- 2017 367 761
- US-B1- 6 235 015
- US-B1- 6 273 885
- US-B1- 8 236 036

## Description

### FIELD OF DISCLOSURE

The present disclosure relates to the technical field of laser hair removal, and in particular, to a handheld home laser hair removal device and system.

### DESCRIPTION OF RELATED ARTS

Laser hair removal devices are known, inter alia, from US 2016/0287333 A1 and US 2009/0204109 A1. At present, a traditional home laser hair removal device uses edge-emitting semiconductor lasers, which emit beams in a direction parallel to a surface of the device's substrate and can operate below 35°C. However, high temperature is one of the main reasons for failures (e.g., edge-emitting semiconductor lasers are unable to operate properly) of edge-emitting semiconductor lasers. Therefore, heat sinks are required to dissipate heat from the edge-emitting semiconductor lasers. In addition, although traditional home laser hair removal devices all use metal treatment heads to cool the user's skin, the temperature of the light treatment area is still high, which tends burn the user's skin during hair removal. Meanwhile, the heat sinks also make loud noise. It can be seen that the above factors all negatively affect the user's experience.

Vertical cavity surface emitting lasers (VCSELs) emit beams in a direction perpendicular to the substrate, and light spots output by the VCSELs are circular. The VCSELs have the characteristics of low voltage and high current, and can operate continuously at 80°C without being easily damaged. The traditional home laser hair removal devices need to switch laser power density level when operating, and the laser parameters are fixed when the traditional home laser hair removal devices are shipped, so that they cannot be customized.

Therefore, in view of the above problems, the present disclosure provides a home laser hair removal device with high temperature tolerance, which alleviates the pain of users during hair removal through a cooling mechanism, while meeting the customized requirements of users.

### SUMMARY OF THE PRESENT INVENTION

The invention is defined in the appended set of claims.

The present disclosure provides a handheld home laser hair removal device to solve problems in the related art. By designing the laser and matching it with a cooling unit, the service life of the handheld home laser hair removal device of the present disclosure can be extended and the user's pain during hair removal can be reduced. In addition, the handheld home laser hair removal device can also meet the user's requirements for different power and different wavelengths.

The handheld home laser hair removal device includes a hair removal body, a laser, an optical waveguide, a heat dissipation unit, a cooling unit, and a controller, wherein the laser, the optical waveguide, the heat dissipation unit, the cooling unit, and the controller are disposed on the hair removal body,
wherein the laser includes a substrate and a plurality of laser chips,
wherein the plurality of laser chips is disposed on the substrate, and emits a laser beam, the laser beam is perpendicular to the substrate,
wherein the optical waveguide is disposed in an optical path of the laser beam, and is configured for shaping the laser beam, and a treatment window is formed on an end surface of the optical waveguide,
wherein the heat dissipation unit includes a radiator, which dissipates heat,
wherein the cooling unit cools the optical waveguide,
wherein the controller is electrically connected to the laser, the heat dissipation unit, and the cooling unit, respectively.

The cooling unit includes a semiconductor cooling sheet and a heat transmitter,
wherein a cooling surface of the semiconductor cooling sheet abuts the optical waveguide, and a heating surface of the semiconductor cooling sheet abuts the heat transmitter,
wherein the heat transmitter is connected to the heat dissipation unit.

The handheld home laser hair removal device includes an optical waveguide holder,
wherein the optical waveguide holder is provided with a fixing slot, the optical waveguide is disposed in the fixing slot, a first surface of the optical waveguide is exposed by the fixing slot, and the cooling surface of the semiconductor cooling sheet abuts the first surface of the optical waveguide.

In an embodiment, the handheld home laser hair removal device includes an optical waveguide cover plate,
wherein the optical waveguide cover plate, along with the optical waveguide holder, fixes the optical waveguide, and the optical waveguide cover plate covers the first surface of the optical waveguide,
wherein the optical waveguide cover plate is provided with a through hole, exposing the heating surface of the semiconductor cooling sheet.

In an embodiment, beams emitted by the plurality of laser chips have the same or different wavelengths;
and/or, wavelengths of the beams emitted by the plurality of laser chips are in a range of 500 nm - 1200 nm;
and/or, the laser is a vertical cavity surface emitting laser (VCSEL);
and/or, the optical waveguide is a sapphire optical waveguide;
and/or, the heat dissipation unit further includes a cooling fan, and the cooling fan has a ventilation surface facing the radiator to cool the radiator.

In an embodiment, the handheld home laser hair removal device includes a function indicator board,
wherein the function indicator board is disposed on the optical waveguide cover plate, and illuminates the optical waveguide; the illuminated optical waveguide can indicate where a hair removal area is.

In an embodiment, the function indicator board includes a plurality of light markers with different colors, which are used to indicate different functions of the handheld home laser hair removal device.

For example, different colors are used to identify the functions of the handheld home laser hair removal device, e.g., blue represents the start of the handheld home laser hair removal device's cooling function when it is in standby mode, pink represents the start of the handheld home laser hair removal device's skin rejuvenation function, and red represents the start of the handheld home laser hair removal device's hair removal function.

In an embodiment, wherein a side of the radiator abuts the substrate;
or, the heat dissipation unit further includes a heat sink, a first side of the heat sink abuts the substrate, and a second side of the heat sink abuts the radiator.

In an embodiment, the handheld home laser hair removal device includes a temperature sensor and/or a skin sensor,
wherein the temperature sensor is disposed on the substrate, and monitors the temperature of the plurality of laser chips,
wherein the skin sensor monitors whether the treatment window is close to a user's skin.

In an embodiment, the handheld home laser hair removal device includes a skin color sensor;
the skin color sensor identifies the color of the user's skin.

In an embodiment, a gap is provided between the plurality of laser chips and the optical waveguide, the width of the gap is in a range of 1 mm - 2 mm, and the uniformity of the laser beams emitted by the laser chips is good.

In an embodiment, the cooling fan is an ultra-quiet cooling fan or a variable speed fan.

The present disclosure also provides a handheld home laser hair removal system, including a handheld home laser hair removal device described above, and a data processing device,
wherein the handheld home hair removal device and/or the data processing device include a photographic lens,
wherein the handheld home hair removal device further includes a first communication module, and the first communication module is electrically connected to the controller,
wherein the data processing device further includes a processor and a second communication module capable of being communicatively connected to the first communication module,
wherein the photographic lens is communicatively connected to the first communication module and the second communication module.

In an embodiment, wherein the processor executes a computer program to implement a processing method, the processing method including:
S1, identifying a user's skin color and hair density from skin photographs to obtain an identification result;
S2, outputting operating parameters of the handheld home laser hair removal device based on the identification result.

In an embodiment, the operating parameters include the peak power of the laser, the pulse-width of the laser beam emitted by the laser, the energy density of the laser beam emitted by the laser, and the wavelengths of the laser beam emitted by the laser.

In an embodiment, the processor can be a single-chip microcomputer, which can be an 8-bit minimum system. The processor can also be selected from different brands and models, or higher digits of controllers or processors.

In an embodiment, the controller further includes a touch screen, through which the parameters of each functional unit of the handheld home laser hair removal device can be adjusted and the handheld home laser hair removal device can be turned on and off.

The present disclosure has the following beneficial effects:
(1) The cooling unit directly cools the optical waveguide, which cools the skin in the treatment area while the treatment window outputs the laser beam with high-energy, so that the pain during hair removal can be greatly reduced and the efficiency of hair removal can be improved.
(2) The laser beam is perpendicular to the substrate, and a cross-section of the laser beam is a circular symmetrical spot, which can easily realize the integration of a high density two-dimensional area array and can output the laser beam with different powers and higher power.
(3) The tissue penetration depths of beams with different wavelengths are different, and by having on the substrate the laser chips that can emit beams with different wavelengths, a corresponding excitation wavelength can be selected according to the user's skin condition, so as to avoid incomplete hair removal or damage to the skin caused by a single wavelength.
(4) The VCSELs can operate continuously at 80°C, and can operate when the ambient temperatures is up to 35°C.
(5) Using the ultra-quiet cooling fan or the variable speed fan, the noise of the cooling system can be as low as 30 dB(A), thereby effectively improving the hair removal experience.
(6) By having an external data processing device, skin color and hair density of the user can be identified, and the operating parameters of the treatment scheme can be calculated through algorithms to achieve customized hair removal;
(7) The handheld home laser hair removal device has an integrated modular design with high integration and small overall size. It can be quickly integrated with different styles of handheld home laser hair removal devices.

### BRIEF DESCRIPTION OF THE DRAWINGS

FIG. 1 is a schematic structural diagram of a handheld home laser hair removal device according to Embodiment 1 of the present disclosure;
FIG. 2 is an exploded view of the handheld home laser hair removal device shown in FIG. 1;
FIG. 3 is a schematic structural diagram of a laser according to Embodiment 1 of the present disclosure;
FIG 4 is a partial exploded view of a handheld home laser hair removal device according to Embodiment 1 of the present disclosure;
FIG. 5 is a schematic structural diagram of a handheld home laser hair removal device according to Embodiment 2 of the present disclosure;
FIG. 6 shows a skin color chart according to Embodiment 3 of the present disclosure.

### Reference Numerals

- 1: Laser
- 11: Substrate
- 12: Laser chips
- 2: Optical waveguide
- 21: Treatment window
- 22: Optical waveguide holder
- 221: Fixing slot
- 23: Optical waveguide cover plate
- 231: Through hole
- 31: Radiator
- 32: Heat sink
- 33: Cooling fan
- 41: Semiconductor cooling sheet
- 42: Heat transmitter
- 5: Function indicator board
- 6: Control board

### DETAILED DESCRIPTION OF THE EMBODIMENTS

The technical solutions in embodiments of the present disclosure will be clearly and completely described below in conjunction with the accompanying drawings in embodiments of the present disclosure. Obviously, the described embodiments are only a part of all embodiments of the present disclosure. Based on the embodiments of the present disclosure, all other embodiments obtained by those skilled in the art without creative labor are within the scope of the present disclosure.

In the description of the present disclosure, it should be noted that the terms "upper/lower end", "inner", "outer", "front end", "rear end", "two ends", "one end", "the other end" indicating orientation or positional relationship are based on the orientation or positional relationship shown in the accompanying drawings, are only for the purpose of facilitating the description of the present disclosure and simplifying the description, and do not indicate or imply that the device or element referred to must have a particular orientation, be constructed and operated in a particular orientation, and therefore they cannot be construed as exclusive limitations of the present disclosure. Furthermore, the terms "first" and "second" are used for descriptive purposes only and are not to be understood as indicating or implying relative importance.

In the description of the present disclosure, it should be noted that, unless otherwise expressly specified and limited, the terms "installation", "set/sleeve", "socket", "connection", etc., should be understood in a broad sense. For example, "connection" can be a fixed connection, a detachable connection, or an integrated connection; it can be a mechanical connection or an electrical connection; it can be directly connected or indirectly connected through an intermediate medium, or it can be a communication between two components. For those skilled in the art, the specific meanings of the above terms in the present disclosure can be understood according to the specific situations.

### Embodiment 1

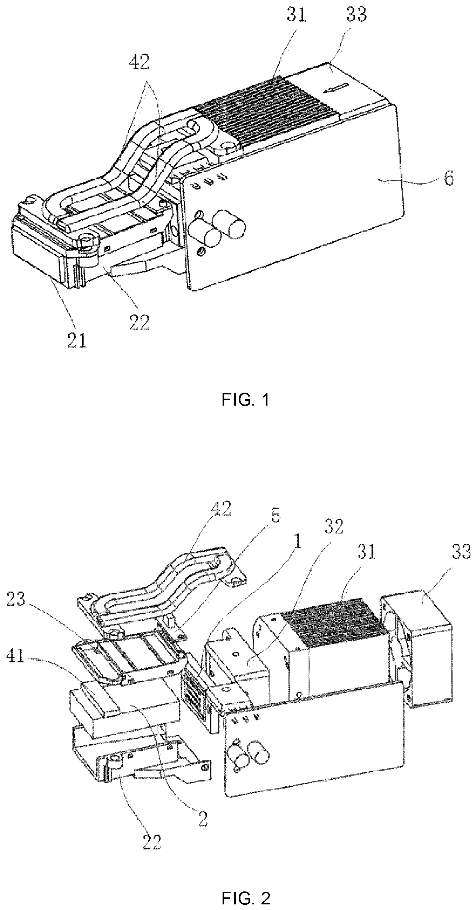

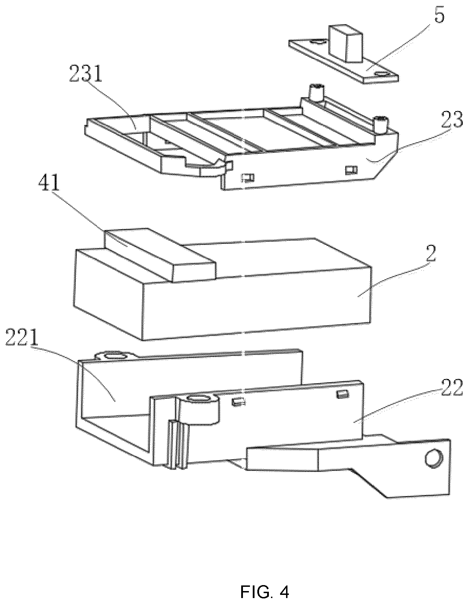

Referring to FIGs. 1-4. A handheld home laser hair removal device includes a hair removal body, a laser 1, an optical waveguide 2, a heat dissipation unit, a cooling unit, and a controller; the laser 1, the optical waveguide 2, the heat dissipation unit, the cooling unit, and the controller are disposed on the hair removal body. The laser 1 includes a substrate 11 and a plurality of laser chips 12; the plurality of the laser chips 12 is disposed on the substrate 11, and emits a laser beam; the laser beam is perpendicular to the substrate 11. The optical waveguide 2 is disposed in an optical path of the laser beam, and is configured for shaping the laser beam; a treatment window 21 is formed on an end surface of the optical waveguide 2; the heat dissipation unit includes a radiator 31, which dissipates heat; the radiator 31 directly abuts the substrate 11. The cooling unit cools the optical waveguide 2; the controller is electrically connected to the laser 1, the heat dissipation unit, and the cooling unit, respectively. The controller and other electrical components are disposed on a control board 6.

In an embodiment, the laser 1 adopts a VCSEL; the VCSEL can operate continuously at 80°C and can operate when the ambient temperature is up to 35°C. An end surface of the optical waveguide 2 is used as the treatment window 21, which directly touches the user's skin, so that the transmittance of the laser optical path is high.

In an embodiment, the optical waveguide 2 is made of sapphire; sapphire is resistant to chemical corrosion, high temperature, has high hardness and good cooling effect. In addition, a surface of the sapphire can be directly used as the treatment window, in which case the transmittance of the laser optical path can be above 90%. The use of sapphire as the optical waveguide for shaping the optical path makes the optical path simple, and the light spot uniform, making it easy to obtain the desired shape of the light spot. In an embodiment, the treatment window is preferably 10 mm × 30 mm.

In an embodiment, because the direction in which the laser beam is emitted is perpendicular to the substrate 11, a thermal conductive base of the laser 1 can be directly attached to the surface of the radiator 31, and no additional transfer heat sinks are required, so that there is a large contact area between the thermal conductive base and the radiator 31, and the heat dissipation efficiency is also high. The above structural design saves a lot of space, which provides space for improving the performance of the whole device and optimizing the appearance of the whole device.

In an embodiment, the plurality of laser chips 12 is disposed on the substrate 11 and emits the laser beam; the direction in which the laser beam is emitted is perpendicular to the substrate 11, which can achieve the integration of a high density two-dimensional area array, so that peak power of the handheld home laser hair removal device can be as high as 100 watts or even kilowatts, and a large energy density can be obtained, significantly improving the hair removal effect. Since the peak power is increased to ensure the hair removal effect, the pulse-width can be greatly reduced, which can effectively reduce the pain during hair removal.

In an embodiment, multiple packaging sites are set up on the substrate 11, the laser chips 12 with the same power or different power can be selected for packaging on the substrate 11, and empty packaging sites can be reserved for subsequent upgrades and transformation according to different requirements of users.

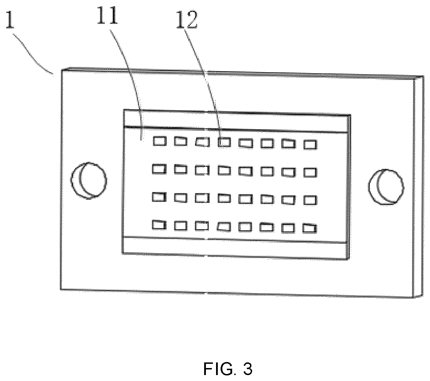

In an embodiment, referring to FIG. 3, the plurality of laser chips 12 are arranged in a matrix of four rows and eight columns, with a row spacing of 3.2 mm and a column spacing of 1.8 mm. In the embodiment, a beam emitted by a single laser chip 12 forms a circular light spot with a divergence angle of 25°, which means the laser beam emitted by the plurality of laser chips 12 is narrow, and the output light spot has good uniformity. In addition, by changing the arrangement of the matrix and the spacing, the plurality of laser chips 12 can output a larger light spot with uniform energy.

In another embodiment, the optical waveguide uses an ordinary photoconductive substance (e.g., glass) as the treatment window. The laser 1 is directly attached to the treatment window (i.e., grass) and the laser beam emitted by the laser 1 does not need to be homogenized and shaped by the optical waveguide 2.

In an embodiment, the laser beam emitted by the plurality of laser chips 12 has the same or different wavelengths; and/or, wavelengths of the beams emitted by the plurality of laser chips 12 are in a range of 500 nm - 1200 nm.

In an embodiment, the laser chips 12 which can emit beams with three wavelengths of 755 nm, 810 nm, and1064 nm are disposed on the substrate 11, so that the laser beam (including one or more beams) with multiple wavelengths can be emitted by the laser chips 12. According to the requirements of users, wavelengths of the beams emitted by the laser chips can be selected.

In an embodiment, the cooling unit includes a semiconductor cooling sheet 41 and a heat transmitter 42. A cooling surface of the semiconductor cooling sheet 41 abuts the optical waveguide 2, and a heating surface of the semiconductor cooling sheet 41 abuts the heat transmitter 42. The heat transmitter 42 is connected to the radiator 31.

In an embodiment, the semiconductor cooling sheet 41 directly cools the optical waveguide 2, and the heat transmitter 42 is a heat pipeline, so that the heat of the semiconductor cooling sheet 42 can be quickly transferred to the radiator 31, and then dissipated into the air through the radiator 31, thereby effectively simplifying the heat dissipation system.

In an embodiment, the optical waveguide 2 is made of sapphire; a surface of the sapphire is used as the treatment window 21, and the semiconductor cooling sheet 41 abuts the sapphire to cool the sapphire, which can lower the temperature of the treatment window 21, effectively cool the user's skin, and significantly reduce the user's pain during hair removal.

In an embodiment, the heat dissipation unit further includes a heat sink 32, a first side of the heat sink 32 abuts the substrate 11, and a second side of the heat sink 32 abuts the radiator 31.

In an embodiment, referring to FIG. 2, the heat sink 32 is used as a holder for the laser 1. For different models of the laser1, only the corresponding heat sink 32 needs to be replaced to achieve universality.

The handheld home laser hair removal device further includes an optical waveguide holder 22; the optical waveguide holder 22 is provided with a fixing slot 221, the optical waveguide 2 is disposed in the fixing slot 221, a first surface of the optical waveguide 2 is exposed by the fixing slot 221, and the cooling surface of the semiconductor cooling sheet 41 abuts the first surface of the optical waveguide 2.

In an embodiment, the handheld home laser hair removal device further includes an optical waveguide cover plate 23. The optical waveguide cover plate 23, along with the optical waveguide holder 22, fixes the optical waveguide 2, and the optical waveguide cover plate 23 covers the first surface of the optical waveguide 2, the optical waveguide cover plate 23 is provided with a through hole 231, exposing the heating surface of the semiconductor cooling sheet 41.

In an embodiment, the optical waveguide cover plate 23 and the optical waveguide holder 22 are tightly fixed by snaps, and form a fixed and closed module along with the laser 1. In addition, the cooling surface of the semiconductor cooling sheet 41 is tightly attached to the first surface of the optical waveguide 2, and the heating surface of the semiconductor cooling sheet 41 is attached to the heat pipeline; the semiconductor cooling sheet 41 and the optical waveguide holder 22 are tightened and fixed by the heat pipeline.

In an embodiment, the handheld home laser hair removal device further includes a function indicator board 5. The function indicator board 5 is disposed on the optical waveguide cover plate 23, and illuminates the entire optical waveguide 2. The illuminated optical waveguide can indicate where a hair removal area is.

In an embodiment, the function indicator board 5 includes a plurality of light markers with different colors, which are used to indicate different functions of the handheld home laser hair removal device. In an embodiment, different colors are used to identify the gears and functions of the handheld home laser hair removal device. For example, blue represents the start of the handheld home laser hair removal device's cooling function when it is in standby mode, pink represents the start of the handheld home laser hair removal device's skin rejuvenation function, and red represents the start of the handheld home laser hair removal device's hair removal function.

In an embodiment, the function indicator board 5 is disposed on a side of the optical waveguide 2 close to the laser 1.

In an embodiment, the heat dissipation unit further includes a cooling fan 33, and the cooling fan 33 is disposed on the radiator 31 to cool the radiator 31.

In an embodiment, the laser 1 is a VCSEL, which can operate continuously at 80 °C. In an embodiment, the cooling fan 33 is a low-noise fan, which can reduce the noise generated by the heat dissipation unit to under 30 dB(A), effectively improving the hair removal experience.

In an embodiment, the handheld home laser hair removal device further includes a temperature sensor, and the temperature sensor is disposed on the substrate and monitors the temperature of the plurality of laser chips 12.

In an embodiment, the cooling fan is a variable speed fan. The variable speed fan and the temperature sensor are connected to the controller respectively. The speed of the variable speed fan can be adjusted according to the temperature of the laser chips 12 measured by the temperature sensor, so that the handheld home laser hair removal device can be used in harsh environments and output high energy.

In an embodiment, the handheld home laser hair removal device further includes a skin sensor, and the skin sensor monitors whether the treatment window is close to the user's skin. Only when the treatment window 21 is in contact with the user's skin, the handheld home laser hair removal device emits beams, to ensure the safety of laser use.

In an embodiment, the handheld home laser hair removal device further includes a skin color sensor, which can identify the skin color of the user.

In an embodiment, a gap is provided between the laser chips 12 and the optical waveguide, and the width of the gap is in a range of 1 mm - 2 mm.

### Embodiment 2

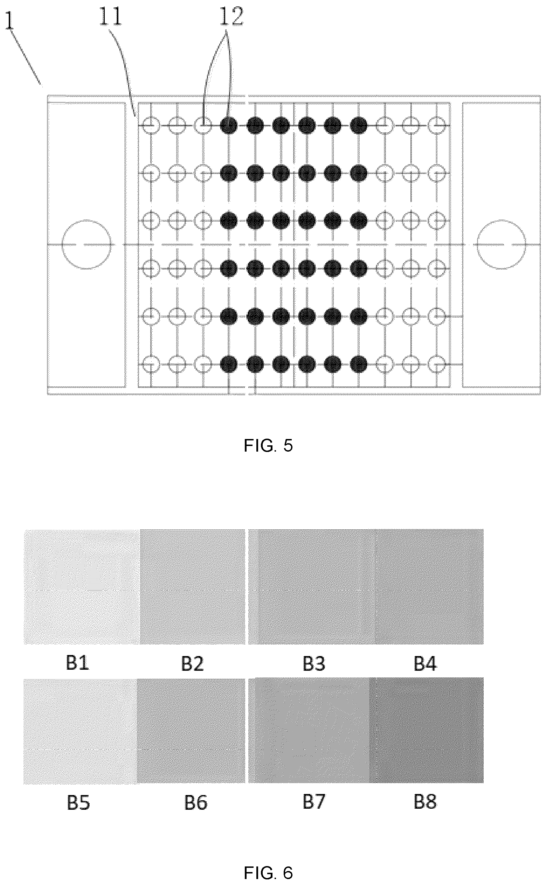

Referring to FIG. 5, there are some black dots and hollow dots on the substrate, where the black dots represent packaged laser chips 12, and the hollow dots represent reserved empty packaged sites. The plurality of laser chips 12 are arranged in a matrix of six rows and six columns, and six rows and three columns of empty packaged sites are arranged on each of two opposing sides of the above matrix. In Embodiment 2, the row spacing is 3.7 mm and the column spacing is 2 mm, which can make the light spot more uniformity as shown by experiments, so that the device can output a uniform light spot without extra homogenization processes.

### Embodiment 3

The present disclosure provides a handheld home laser hair removal system, including a handheld home laser hair removal device described above, and a data processing device; the handheld home hair removal device further includes a first communication module, and the first communication module is electrically connected to the controller. The data processing device further includes a photographic lens, a processor, and a second communication module capable of being communicatively connected to the first communication module; the processor is communicatively connected to the photographic lens and the second communication module. The photographic lens is used to take pictures of the user's skin to obtain skin photographs, and transmits the skin photographs to the processor for processing; the second communication module receives a processing result of the processor and transmits the processing result to the first communication module; the first communication module further transmits the processing result to the controller.

In an embodiment, the processor executes a computer program to implement a processing method, the processing method includes:
S1, identifying a user's skin color and hair density from the skin photographs to obtain an identification result;
S2, outputting operating parameters of the handheld home laser hair removal device based on the identification result.

In some embodiments, the operating parameters include peak power of the laser, pulse-width of the laser beam emitted by the laser, energy density of the laser beam emitted by the laser, and wavelengths of the laser beam emitted by the laser.

In an embodiment, the data processing device is a mobile phone or a tablet computer, etc., and the first communication module and the second communication module are wireless data transmission modules, such as WiFi modules, Bluetooth modules, infrared wireless modules, etc.

Applications are installed in the mobile phone and the tablet computer, and photos of multiple (e.g., the quantity is A) skin types, multiple (e.g., the quantity is B) skin colors, and multiple (e.g., the quantity is C) hair densities are stored in the memory or remote servers. The above A, B, and C are integers, and each parameter corresponds to the scheme of each parameter of different hair removal device.

Firstly, the user's skin is taken pictures of with a mobile phone to get skin photographs, and the skin photographs are compared with the skin types, skin colors, and hair densities (whose unit is hairs per square centimeter) that are stored in the memory by the algorithm, and different schemes with the appropriate operating parameters are transmitted to the controller through the wireless data transmission module. The operating parameters include the peak power of the laser, the pulse-width of the laser beam emitted by the laser, the energy density of the laser beam emitted by the laser, and the wavelengths of the laser beam emitted by the laser.

The controller further includes a touch screen through which parameters of each functional unit of the handheld home laser hair removal device can be adjusted and the handheld home laser hair removal device can be turned on and off.

In an embodiment, referring to FIG. 6, eight skin colors are stored. There are eight types of hair density, which are five hairs per square centimeter, ten hairs per square centimeter, fifteen hairs per square centimeter, twenty hairs per square centimeter, twenty-five hairs per square centimeter, thirty hairs per square centimeter, thirty-five hairs per square centimeter, forty hairs per square centimeter. In an embodiment, the above eight skin colors and eight types of hair density correspond to 64 schemes with different operating parameters.

In an embodiment, for the B1 skin color in FIG. 6, the user's hair density is approximately ten hairs per square centimeter, an output power of the laser 1 is 100 watts, and the wavelength of the laser beam emitted by the laser 1 is 810 nm.

It should be noted that, terms such as "first", "second" are only used to distinguish one entity or operation from another entity or operation, and do not necessarily require or imply any such actual relationship or order between these entities or operations. Moreover, the terms "including", "containing" or any other variant thereof is intended to cover non-exclusive inclusion, so that a process, method, item or device that includes a series of elements not only includes those elements, but also includes other elements that are not explicitly listed or inherent in such a process, method, item or device. In the absence of further limitations, the element limited by the phrase "including a..." does not exclude another identical element in the process, method, item or device that includes the element.

Although embodiments of the present disclosure have been shown and described, various changes, modifications, substitutions and alterations can be made by those skilled in the art without departing from the principles of the present disclosure.

## Claims

1. A handheld home laser hair removal device, comprising: a hair removal body, a laser (1), an optical waveguide (2), an optical waveguide holder (22), a heat dissipation unit, a cooling unit, and a controller, wherein the laser (1), the optical waveguide (2), the heat dissipation unit, the cooling unit, and the controller are disposed on the hair removal body,
wherein the laser (1) comprises a substrate (11) and a plurality of laser chips (12),
wherein the plurality of laser chips (12) is disposed on the substrate (11), and is configured to emit laser beams being perpendicular to the substrate (11),
wherein the optical waveguide (2) is disposed in an optical path of the laser beam, and is configured for shaping the laser beam, and a treatment window (21) is formed on an end surface of the optical waveguide (2), wherein the optical waveguide holder (22) comprises a fixing slot (221) receiving the optical waveguide (2),
wherein the heat dissipation unit comprises a radiator (31) configured to dissipate heat,
wherein the cooling unit is configured to cool the optical waveguide (2), and comprises a semiconductor cooling sheet (41), wherein a cooling surface of the semiconductor cooling sheet (41) abuts a first surface of the optical waveguide (2) exposed by the fixing slot (221),
wherein the controller is electrically connected to the laser (1), the heat dissipation unit, and the cooling unit, respectively.

2. The handheld home laser hair removal device according to claim 1, wherein the cooling unit further comprises a heat transmitter (42),
wherein a heating surface of the semiconductor cooling sheet (41) abuts the heat transmitter (42),
wherein the heat transmitter (42) is connected to the heat dissipation unit.

3. The handheld home laser hair removal device according to claim 1, further comprising an optical waveguide cover plate (23),
wherein the optical waveguide cover plate (23), along with the optical waveguide holder (22), fixes the optical waveguide (2), and the optical waveguide cover plate (23) covers the first surface of the optical waveguide,
wherein the optical waveguide cover plate (23) is provided with a through hole (231), exposing the heating surface of the semiconductor cooling sheet (41).

4. The handheld home laser hair removal device according to claim 1, wherein laser beams emitted by the plurality of laser chips (12) have the same or different wavelengths;
and/or, wavelengths of the beams emitted by the plurality of laser chips (12) are in a range of 500 nm - 1200 nm;
and/or, the laser (1) is a vertical cavity surface emitting laser, VCSEL;
and/or, the optical waveguide (2) is a sapphire optical waveguide;
and/or, the heat dissipation unit further comprises a cooling fan (33), and the cooling fan (33) has a ventilation surface facing the radiator (31) to cool the radiator (31).

5. The handheld home laser hair removal device according to claim 3, further comprising a function indicator board (5),
wherein the function indicator board (5) is disposed on the optical waveguide cover plate (23), and configured to illuminate the optical waveguide through the optical waveguide cover plate (23);
and/or, the function indicator board (5) comprises a plurality of light markers with different colors.

6. The handheld home laser hair removal device according to any one of claims 1-5, wherein a side of the radiator (31) abuts the substrate (11);
or, the heat dissipation unit further comprises a heat sink (32), a first side of the heat sink (32) abuts the substrate (11), and a second side of the heat sink (32) abuts the radiator (31).

7. The handheld home laser hair removal device according to claim 6, further comprising: a temperature sensor and/or a skin sensor,
wherein the temperature sensor is disposed on the substrate, and is configured to monitor the temperature of the plurality of laser chips,
wherein the skin sensor is configured to monitor whether the treatment window is close to a user's skin.

8. A handheld home laser hair removal system, comprising a handheld home laser hair removal device according to any one of claims 1-7, and a data processing device,
wherein the handheld home hair removal device and/or the data processing device comprise a photographic lens,
wherein the handheld home hair removal device further comprises a first communication module, and the first communication module is electrically connected to the controller,
wherein the data processing device further comprises a processor and a second communication module capable of being communicatively connected to the first communication module,
wherein skin photographs taken using the photographic lens are transmitted to the processor for processing, and the second communication module is configured to receive a processing result of the processor and transmit the processing result to the first communication module.

9. The handheld home laser hair removal system according to claim 8, wherein the processor is configured to execute a computer program to implement a processing method, the processing method comprising:
S1, identifying a user's skin color and hair density from the skin photographs to obtain an identification result;
S2, outputting operating parameters of the handheld home laser hair removal device, based on the identification result.

## Patentansprüche

1. Ein handgehaltenes Laser-Haarentfernungsgerät für den Heimgebrauch, umfassend: einen Haarentfernungskörper, einen Laser (1), einen optischen Wellenleiter (2), einen optischen Wellenleiterhalter (22), eine Wärmeableiteinheit, eine Kühleinheit und einen Controller, wobei der Laser (1), der optische Wellenleiter (2), die Wärmeableiteinheit, die Kühleinheit und der Controller an dem Haarentfernungskörper angeordnet sind, wobei der Laser (1) ein Substrat (11) und eine Mehrzahl von Laserchips (12) umfasst, wobei die Mehrzahl von Laserchips (12) auf dem Substrat (11) angeordnet ist und dazu konfiguriert ist, Laserstrahlen zu emittieren, die senkrecht zu dem Substrat (11) sind, wobei der optische Wellenleiter (2) in einem optischen Pfad des Laserstrahls angeordnet ist und zum Formen des Laserstrahls konfiguriert ist, und ein Behandlungsfenster (21) an einer Endfläche des optischen Wellenleiters (2) ausgebildet ist, wobei der optische Wellenleiterhalter (22) einen Befestigungsschlitz (221) umfasst, der den optischen Wellenleiter (2) aufnimmt, wobei die Wärmeableiteinheit einen Radiator (31) umfasst, der dazu konfiguriert ist, Wärme abzuführen, wobei die Kühleinheit dazu konfiguriert ist, den optischen Wellenleiter (2) zu kühlen, und ein Halbleiter-Kühlblech (41) umfasst, wobei eine Kühloberfläche des Halbleiter-Kühlblechs (41) an einer ersten Oberfläche des optischen Wellenleiters (2) anliegt, die durch den Befestigungsschlitz (221) freigelegt ist, wobei der Controller jeweils elektrisch mit dem Laser (1), der Wärmeableiteinheit und der Kühleinheit verbunden ist.

2. Das handgehaltene Laser-Haarentfernungsgerät für den Heimgebrauch nach Anspruch 1, wobei die Kühleinheit ferner einen Wärmeüberträger (42) umfasst, wobei eine Heizoberfläche des Halbleiter-Kühlblechs (41) an dem Wärmeüberträger (42) anliegt, wobei der Wärmeüberträger (42) mit der Wärmeableiteinheit verbunden ist.

3. Das handgehaltene Laser-Haarentfernungsgerät für den Heimgebrauch nach Anspruch 1, ferner umfassend eine optische Wellenleiter-Abdeckplatte (23), wobei die optische Wellenleiter-Abdeckplatte (23) zusammen mit dem optischen Wellenleiterhalter (22) den optischen Wellenleiter (2) fixiert und die optische Wellenleiter-Abdeckplatte (23) die erste Oberfläche des optischen Wellenleiters abdeckt, wobei die optische Wellenleiter-Abdeckplatte (23) mit einem Durchgangsloch (231) versehen ist, das die Heizoberfläche des Halbleiter-Kühlblechs (41) freilegt.

4. Das handgehaltene Laser-Haarentfernungsgerät für den Heimgebrauch nach Anspruch 1, wobei die von der Mehrzahl von Laserchips (12) emittierten Laserstrahlen die gleichen oder unterschiedliche Wellenlängen haben; und/oder Wellenlängen der von der Mehrzahl von Laserchips (12) emittierten Strahlen in einem Bereich von 500 nm - 1200 nm liegen; und/oder der Laser (1) ein vertikaler Resonator-Oberflächenemissionslaser, VCSEL, ist; und/oder der optische Wellenleiter (2) ein Saphir-Wellenleiter ist; und/oder die Wärmeableiteinheit ferner einen Kühlventilator (33) umfasst, und der Kühlventilator (33) eine Belüftungsfläche aufweist, die dem Radiator (31) zugewandt ist, um den Radiator (31) zu kühlen.

5. Das handgehaltene Laser-Haarentfernungsgerät für den Heimgebrauch nach Anspruch 3, ferner umfassend eine Funktionsanzeigeplatine (5), wobei die Funktionsanzeigeplatine (5) an der optischen Wellenleiter-Abdeckplatte (23) angeordnet ist und dazu konfiguriert ist, den optischen Wellenleiter durch die optische Wellenleiter-Abdeckplatte (23) zu beleuchten; und/oder die Funktionsanzeigeplatine (5) eine Mehrzahl von Leuchtmarkierungen mit unterschiedlichen Farben umfasst.

6. Das handgehaltene Laser-Haarentfernungsgerät für den Heimgebrauch nach einem der Ansprüche 1-5, wobei eine Seite des Radiators (31) an dem Substrat (11) anliegt; oder die Wärmeableiteinheit ferner eine Wärmesenke (32) umfasst, wobei eine erste Seite der Wärmesenke (32) an dem Substrat (11) anliegt und eine zweite Seite der Wärmesenke (32) an dem Radiator (31) anliegt.

7. Das handgehaltene Laser-Haarentfernungsgerät für den Heimgebrauch nach Anspruch 6, ferner umfassend: einen Temperatursensor und/oder einen Hautsensor, wobei der Temperatursensor auf dem Substrat angeordnet ist und dazu konfiguriert ist, die Temperatur der Mehrzahl von Laserchips zu überwachen, wobei der Hautsensor dazu konfiguriert ist, zu überwachen, ob das Behandlungsfenster nahe an der Haut eines Benutzers ist.

8. Ein handgehaltenes Laser-Haarentfernungssystem für den Heimgebrauch, umfassend ein handgehaltenes Laser-Haarentfernungsgerät für den Heimgebrauch nach einem der Ansprüche 1-7 und eine Datenverarbeitungsvorrichtung, wobei das handgehaltene Heim-Haarentfernungsgerät und/oder die Datenverarbeitungsvorrichtung ein photographisches Objektiv umfasst/umfassen, wobei das handgehaltene Heim-Haarentfernungsgerät ferner ein erstes Kommunikationsmodul umfasst und das erste Kommunikationsmodul elektrisch mit dem Controller verbunden ist, wobei die Datenverarbeitungsvorrichtung ferner einen Prozessor und ein zweites Kommunikationsmodul umfasst, das dazu geeignet ist, kommunikativ mit dem ersten Kommunikationsmodul verbunden zu sein, wobei unter Verwendung des photographischen Objektivs aufgenommene Hautfotografien zur Verarbeitung an den Prozessor übertragen werden und das zweite Kommunikationsmodul dazu konfiguriert ist, ein Verarbeitungsergebnis des Prozessors zu empfangen und das Verarbeitungsergebnis an das erste Kommunikationsmodul zu übertragen.

9. Das handgehaltene Laser-Haarentfernungssystem für den Heimgebrauch nach Anspruch 8, wobei der Prozessor dazu konfiguriert ist, ein Computerprogramm auszuführen, um ein Verarbeitungsverfahren zu implementieren, wobei das Verarbeitungsverfahren Folgendes umfasst: S1, Identifizieren der Hautfarbe und Haardichte eines Benutzers aus den Hautfotografien, um ein Identifikationsergebnis zu erhalten; S2, Ausgeben von Betriebsparametern des handgehaltenen Laser-Haarentfernungsgeräts für den Heimgebrauch, basierend auf dem Identifikationsergebnis.

## Revendications

1. Un dispositif portatif d'épilation laser à domicile, comprenant : un corps d'épilation, un laser (1), un guide d'ondes optique (2), un porte-guide d'ondes optique (22), une unité de dissipation thermique, une unité de refroidissement et un contrôleur, le laser (1), le guide d'ondes optique (2), l'unité de dissipation thermique, l'unité de refroidissement et le contrôleur étant disposés sur le corps d'épilation, le laser (1) comprenant un substrat (11) et une pluralité de puces laser (12), la pluralité de puces laser (12) étant disposée sur le substrat (11) et étant configurée pour émettre des faisceaux laser perpendiculaires au substrat (11), le guide d'ondes optique (2) étant disposé dans un trajet optique du faisceau laser et étant configuré pour façonner le faisceau laser, et une fenêtre de traitement (21) étant formée sur une surface d'extrémité du guide d'ondes optique (2), le porte-guide d'ondes optique (22) comprenant une rainure de fixation (221) recevant le guide d'ondes optique (2), l'unité de dissipation thermique comprenant un radiateur (31) configuré pour dissiper la chaleur, l'unité de refroidissement étant configurée pour refroidir le guide d'ondes optique (2) et comprenant une plaque de refroidissement à semi-conducteur (41), une surface de refroidissement de la plaque de refroidissement à semi-conducteur (41) étant en appui contre une première surface du guide d'ondes optique (2) exposée par la rainure de fixation (221), le contrôleur étant électriquement connecté respectivement au laser (1), à l'unité de dissipation thermique et à l'unité de refroidissement.

2. Le dispositif portatif d'épilation laser à domicile selon la revendication 1, dans lequel l'unité de refroidissement comprend en outre un transmetteur de chaleur (42), une surface de chauffage de la plaque de refroidissement à semi-conducteur (41) étant en appui contre le transmetteur de chaleur (42), le transmetteur de chaleur (42) étant connecté à l'unité de dissipation thermique.

3. Le dispositif portatif d'épilation laser à domicile selon la revendication 1, comprenant en outre une plaque de recouvrement de guide d'ondes optique (23), la plaque de recouvrement de guide d'ondes optique (23), conjointement avec le porte-guide d'ondes optique (22), fixant le guide d'ondes optique (2), et la plaque de recouvrement de guide d'ondes optique (23) recouvrant la première surface du guide d'ondes optique, la plaque de recouvrement de guide d'ondes optique (23) étant pourvue d'un trou traversant (231), exposant la surface de chauffage de la plaque de refroidissement à semi-conducteur (41).

4. Le dispositif portatif d'épilation laser à domicile selon la revendication 1, dans lequel des faisceaux laser émis par la pluralité de puces laser (12) ont les mêmes ou différentes longueurs d'onde ; et/ou des longueurs d'onde des faisceaux émis par la pluralité de puces laser (12) sont dans une plage de 500 nm - 1200 nm ; et/ou le laser (1) est un laser à émission de surface à cavité verticale, VCSEL ; et/ou le guide d'ondes optique (2) est un guide d'ondes optique en saphir ; et/ou l'unité de dissipation thermique comprend en outre un ventilateur de refroidissement (33), et le ventilateur de refroidissement (33) a une surface de ventilation faisant face au radiateur (31) pour refroidir le radiateur (31).

5. Le dispositif portatif d'épilation laser à domicile selon la revendication 3, comprenant en outre une carte indicatrice de fonction (5), la carte indicatrice de fonction (5) étant disposée sur la plaque de recouvrement de guide d'ondes optique (23) et étant configurée pour illuminer le guide d'ondes optique à travers la plaque de recouvrement de guide d'ondes optique (23) ; et/ou la carte indicatrice de fonction (5) comprend une pluralité de marqueurs lumineux de différentes couleurs.

6. Le dispositif portatif d'épilation laser à domicile selon l'une quelconque des revendications 1-5, dans lequel un côté du radiateur (31) est en appui contre le substrat (11) ; ou l'unité de dissipation thermique comprend en outre un dissipateur thermique (32), un premier côté du dissipateur thermique (32) étant en appui contre le substrat (11), et un deuxième côté du dissipateur thermique (32) étant en appui contre le radiateur (31).

7. Le dispositif portatif d'épilation laser à domicile selon la revendication 6, comprenant en outre : un capteur de température et/ou un capteur de peau, le capteur de température étant disposé sur le substrat et étant configuré pour surveiller la température de la pluralité de puces laser, le capteur de peau étant configuré pour surveiller si la fenêtre de traitement est proche de la peau d'un utilisateur.

8. Un système portatif d'épilation laser à domicile, comprenant un dispositif portatif d'épilation laser à domicile selon l'une quelconque des revendications 1-7 et un dispositif de traitement de données, le dispositif portatif d'épilation à domicile et/ou le dispositif de traitement de données comprenant une lentille photographique, le dispositif portatif d'épilation à domicile comprenant en outre un premier module de communication, et le premier module de communication étant électriquement connecté au contrôleur, le dispositif de traitement de données comprenant en outre un processeur et un second module de communication capable d'être connecté en communication avec le premier module de communication, des photographies de peau prises à l'aide de la lentille photographique étant transmises au processeur pour traitement, et le second module de communication étant configuré pour recevoir un résultat de traitement du processeur et transmettre le résultat de traitement au premier module de communication.

9. Le système portatif d'épilation laser à domicile selon la revendication 8, dans lequel le processeur est configuré pour exécuter un programme informatique afin de mettre en œuvre une méthode de traitement, la méthode de traitement comprenant : S1, identifier une couleur de peau et une densité de poils d'un utilisateur à partir des photographies de peau pour obtenir un résultat d'identification ; S2, produire des paramètres de fonctionnement du dispositif portatif d'épilation laser à domicile, sur la base du résultat d'identification.
